# EUROPEAN PATENT APPLICATION

(11) **EP 4 464 337 A1**
(43) Date of publication of application: **20.11.2024**
(21) Application number: 23740048.6
(22) Date of filing: 11.01.2023
(51) Int. Cl.: A61K 47/68, C07K 16/28, A61K 31/506, A61P 35/00

(54) **COMBINED USE OF ANTI-TROP-2 ANTIBODY-DRUG CONJUGATE AND OTHER THERAPEUTIC AGENTS**

(30) Priority: 13.01.2022 CN 202210036236
(71) Applicant: Sichuan Kelun-Biotech Biopharmaceutical Co., Ltd., Chengdu, Sichuan 611138 (CN)
(72) Inventor: JIN, Xiaoping, Chengdu, Sichuan 611138 (CN); DIAO, Yina, Chengdu, Sichuan 611138 (CN); GUO, Qi, Chengdu, Sichuan 611138 (CN); LIU, Gesha, Chengdu, Sichuan 611138 (CN); LU, Lian, Chengdu, Sichuan 611138 (CN); WEI, Yujun, Chengdu, Sichuan 611138 (CN); YIN, Wenguo, Chengdu, Sichuan 611138 (CN); WANG, Suxia, Chengdu, Sichuan 611138 (CN); PAN, Yang, Chengdu, Sichuan 611138 (CN); GE, Junyou, Chengdu, Sichuan 611138 (CN)
(74) Representative: Cabinet Nony
(86) International application number: PCT/CN2023/071796
(87) International publication number: WO 2023/134706

(57) **Abstract**

A combination of an anti-TROP-2 antibody-drug conjugate and anti-PD-L1 antibody or other chemotherapeutic drugs such as Carboplatin or Cisplatin, and an application of the combination in the preparation of drugs for preventing and/or treating tumor diseases, wherein the anti-TROP-2 antibody may be Sacituzumab.

## Description

The present application is based on the application with CN application number 202210036236.5 and the filing date of January 13, 2022, and claims its priority. The content disclosed in the CN application is hereby incorporated into the present application in its entirety.

### Technical Field

The present invention belongs to the field of tumor treatment and molecular immunology and relates to a combined use of anti-TROP-2 antibody-drug conjugate and other therapeutic agents, for example, a combined use with a PD-L1 inhibitor and a chemotherapeutic agent, in the manufacture of a medicament for the prevention and/or treatment of tumor diseases.

### Background Art

Malignant tumors have become a common and frequently-occurring disease in China, which seriously threatens human life and health. A research report from the International Agency for Research on Cancer shows that the new cases and deaths from malignant tumors in China account for approximately 21.8% and 27% those of the world, respectively. The main treatment method for advanced malignant tumors is still chemotherapy, but chemotherapeutic agents can not only kill tumor cells, but also attack normal cells of the human body indiscriminately, thereby causing serious side effects.

In recent years, the application of immune checkpoint inhibitors (ICIs) leads medicine scaling into an era of immune drug therapy, and ICIs have changed the clinical practice of tumor treatment. At present, the use of ICIs has evolved from the initial second-line treatment for most tumor types to the current first-line treatment for some tumor types, and even the postoperative adjuvant treatment for a few tumor types and changed from monotherapy to combination therapy. Clinically, ICIs are usually combined with chemotherapy to enhance the efficacy and promote ICIs to induce effective anti-tumor responses in a wider patient population, and their synergistic effects have been confirmed in clinical studies.

It is reported that camptothecin drugs can activate the immune system and have the potential to synergize with ICIs. Camptothecin (CPT), Camptothecin sodium (CPT-Na), Irinotecan hydrochloride (CPT-11) and Topotecan hydrochloride (TPT) can effectively activate dendritic cells through nuclear factor κB (NF-xB)-related pathways, upregulating the expression of major histocompatibility complex class II (MHC-II), CD40 and CD80 on their surface (Mizumoto et al., 2005). In a mouse breast cancer model, it was further observed that CPT-11 combined with anti-PD-L1 could effectively promote the increase of infiltrating and proliferating CD8+ T cells (Ki67+CD8+ T cells) in draining lymph nodes and tumors and showed a significantly superior anti-tumor effect in comparison with monotherapy (Iwai et al., 2018). In another study, CPT-11 liposome could stimulate human melanoma cells to regulate the expression of relevant genes through the TP53 pathway, thereby effectively activating T cell-mediated immune responses to kill tumors, and the combination thereof with anti-PD-1 or anti-PD-L1 antibody could effectively prolong the survival of mice bearing colon cancer (McKenzie et al., 2018). Moreover, CPT-11 and its active metabolite SN38 were further proved to have the ability to induce immunogenic cell death (ICD), and when it was used in combination with anti-PD-1 and anti-PD-L1 monoclonal antibodies, synergistic anti-tumor effects were observed in both mouse pancreatic cancer and melanoma models, respectively, (Liu et al., 2021, Gong et al., 2020).

For a long time, selectively killing tumor cells and minimizing damage to normal cells have been the development trend of cancer therapy. In recent years, studies have discovered that therapeutic antibodies can be linked to biologically active molecules to form antibody-drug conjugates (ADCs). ADC drugs are complexes formed by conjugating individual antibodies with small molecule cytotoxic drugs through linkers. After ADC drugs enter the blood, their antibody components specifically bind to tumor cell surface antigens, enter the cells through endocytosis, and release cytotoxic drugs to kill the tumor cells. ADC drugs directly target tumor cells, thereby greatly reducing the toxic and side effects of chemotherapeutic agents on other normal cells and reducing the occurrence of systemic adverse events. Since the combined use of ICIs and chemotherapeutic agents that activate the immune system can synergistically enhance the anti-tumor immune response and kill tumors, the ADCs with immunomodulatory effects combined with ICIs may not only achieve synergistic effect but may also further overcome the current limitations of ICIs or chemotherapeutic agents. For example, in a combination therapy of ICIs with chemotherapy, it can avoid the myelosuppression caused by toxin molecules, and the peripheral immune tolerance and autoimmune complications induced by the activation of immune system by toxin molecules (Bauzon et al., 2019).

### Contents of the present invention

The present invention provides a method, pharmaceutical composition, use and kit for treating a cancer by using a combination of therapeutic agents, such as a combination of an antibody-drug conjugate and an additional therapeutic agent.

One aspect of the present invention provides use of an anti-TROP-2 antibody-drug conjugate and an additional therapeutic agent in the manufacture of a medicament for treating a cancer in a subj ect.

In some embodiments, the additional therapeutic agent is one or more selected from the group consisting of an anti-PD-L1 antibody or an antigen-binding fragment thereof and a chemotherapeutic agent.

In some embodiments, the use comprises administering the subject the anti-TROP-2 antibody-drug conjugate, and the anti-PD-L1 antibody or antigen-binding fragment thereof.

In some embodiments, the anti-TROP-2 antibody-drug conjugate comprises a camptothecin drug and an anti-TROP-2 antibody or an antigen-binding fragment thereof, and the anti-TROP-2 antibody or antigen-binding fragment thereof comprises a heavy chain variable region and a light chain variable region, wherein the heavy chain variable region comprises a HCDR1 as set forth in SEQ ID No.: 1, a HCDR2 as set forth in SEQ ID No.: 2 and a HCDR3 as set forth in SEQ ID No.: 3, and the light chain variable region comprises a LCDR1 as set forth in SEQ ID No.: 4, a LCDR2 as set forth in SEQ ID No.: 5 and a LCDR3 as set forth in SEQ ID No.: 6.

In some embodiments, the anti-PD-L1 antibody or antigen-binding fragment thereof is one or more selected from the group consisting of Durvalumab, Atezolizumab, and Envafolimab.

In some embodiments, the anti-PD-L1 antibody or antigen-binding fragment thereof comprises a heavy chain variable region and a light chain variable region, wherein the heavy chain variable region comprises a HCDR1 as set forth in SEQ ID No.: 7, a HCDR2 as set forth in SEQ ID No.: 8 and a HCDR3 as set forth in SEQ ID No.: 9, and the light chain variable region comprises a LCDR1 as set forth in SEQ ID No.: 10, a LCDR2 as set forth in SEQ ID No.: 11 and a LCDR3 as set forth in SEQ ID No.: 12.

In some embodiments, the use further comprises administering a chemotherapeutic agent to the subj ect.

In some embodiments, the chemotherapeutic drug is selected from platinum drugs.

In some embodiments, the platinum drug is selected from the group consisting of Cisplatin, Carboplatin, Sulfatodiaminocyctohexane Platinum, Nedaplatin, Oxaliplatin, Lobaplatin, Satraplatin, Miboplatin, Enloplatin, Iproplatin or Dicycloplatin.

In some embodiments, the anti-TROP-2 antibody comprises a heavy chain as set forth in SEQ ID No.: 13 and a light chain as set forth in SEQ ID No.: 14.

In some embodiments, the anti-PD-L1 antibody or antigen-binding fragment thereof comprises a heavy chain variable region as set forth in SEQ ID No.: 15 and a light chain variable region as set forth in SEQ ID No.: 16.

In some embodiments, the anti-PD-L1 antibody or antigen-binding fragment thereof comprises a mutation in the amino acid sequence of the anti-PD-L1 antibody of the present invention. The variant amino acid sequence is identical to a reference sequence except for having a substitution, deletion and/or addition of one, two, three, four or five amino acids.

In some embodiments, the substitution, deletion and/or addition is located in a CDR. In some embodiments, the substitution, deletion, and/or addition is located in a framework region.

In some embodiments, the substitution of one, two, three, four or five amino acids is a conservative substitution.

In some embodiments, the anti-PD-L1 antibody or antigen-binding fragment thereof has a V_{L} domain with a sequence homology of at least 95%, 90%, 85%, 80%, 75% or 50% to one of the V_{L} domains of the anti-PD-L1 antibody of the present invention, and exhibits specific binding to PD-L 1.

In some embodiments, the anti-PD-L1 antibody or antigen-binding fragment thereof has a V_{H} domain with a sequence homology of at least 95%, 90%, 85%, 80%, 75% or 50% to one of the V_{H} domains of the anti-PD-L1 antibody of the present invention, and exhibits specific binding to PD-L 1.

In some embodiments, the anti-PD-L1 antibody or antigen-binding fragment thereof has a V_{L} domain with a substitution, deletion and/or addition of up to 1, 2, 3, 4, 5 or more amino acids as compared to one of the V_{L} domains of the anti-PD-L1 antibody of the present invention, and exhibits specific binding to PD-L 1.

In some embodiments, the anti-PD-L1 monoclonal antibody or antigen-binding fragment thereof has a V_{H} domain with a substitution, deletion and/or addition of up to 1, 2, 3, 4, 5 or more amino acids as compared to one of the V_{H} domains of the anti-PD-L1 antibody of the present invention, and exhibits specific binding to PD-L1.

In some embodiments, the anti-PD-L1 antibody or antigen-binding fragment thereof has a V_{L} domain with a substitution, deletion and/or addition of up to 1, 2, 3, 4, 5 or more amino acids as compared to one of the V_{L} domains of the anti-PD-L1 antibody of the present invention, and a V_{H} domain with a substitution, deletion and/or addition of up to 1, 2, 3, 4, 5 or more amino acids as compared to one of the V_{H} domains of the anti-PD-L1 antibody of the present invention, and exhibits specific binding to PD-L 1.

Generally, the variant of the anti-PD-L1 monoclonal antibody and antigen-binding fragment thereof disclosed in the present invention has an amino acid sequence with an amino acid sequence identity of at least 75%, more preferably at least 80%, more preferably at least 85%, more preferably at least 90%, most preferably at least 95%, 98% or 99% as compared to an amino acid sequence of a reference antibody or antigen-binding fragment (e.g., a heavy chain, light chain, V_{H}, V_{L}, or humanized sequence). The terminology "identity" or "homology" in the present invention is defined as percentage of the amino acid residues in a candidate sequence that are identical to a reference sequence, if necessary, after aligning the sequences and introducing gaps to achieve maximum percent sequence identity, and not considering any conservative substitutions as part of sequence identity. N-terminus, C-terminus, or internal extensions, deletions, or insertions of antibody sequences shall not be interpreted as affecting sequence identity or homology.

In some embodiments, the anti-PD-L1 monoclonal antibody is a human antibody. In other embodiments, the anti-PD-L1 monoclonal antibody is a humanized antibody.

In some embodiments, the antibody-drug conjugate has the following chemical structure: 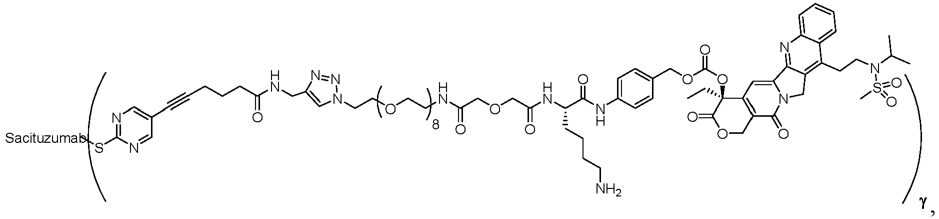 wherein γ represents the average number of drug-linker units conjugated to each antibody molecule in the antibody-drug conjugate, and the average number is in the range of 6 to 10. In some embodiments, the average number of γ is between 6 and 9. In some embodiments, the average number of γ is between 6 and 8, for example about 6.1, 6.2, 6.3, 6.4, 6.5, 6.6, 6.7, 6.8, 6.9, 7, 7.1, 7.2, 7.3, 7.4, 7.5, 7.6, 7.7, 7.8 or 7.9.

In some embodiments, the cancer is a solid tumor or a non-solid tumor; for example, selected from the group consisting of esophageal cancer (e.g., esophageal adenocarcinoma and squamous cell esophageal carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, and thyroid cancer.

In some embodiments, the cancer is selected from the group consisting of lung cancer, breast cancer, and epithelial ovarian cancer.

In some embodiments, the cancer is non-small cell lung cancer (NSCLC) or triple negative breast cancer (TNBC).

In some embodiments, the non-small cell lung cancer is selected from EGFR wild-type or mutated non-small cell lung cancer.

In some embodiments, the non-small cell lung cancer is an advanced/metastatic non-small cell lung cancer.

In some embodiments, the non-small cell lung cancer is an advanced non-small cell lung cancer that has not been treated with a systemic therapy or has received at most a first-line chemotherapy.

In some embodiments, the non-small cell lung cancer is an ALK rearrangements-negative non-small cell lung cancer.

In some embodiments, the EGFR mutation is single mutation or compound mutation.

In some embodiments, the breast cancer is selected from the group consisting of triple negative breast cancer, HR+/HER2- breast cancer, HR-/HER2+ breast cancer, HR+/HER2+ breast cancer, and BRCA+ breast cancer.

In some embodiments, the breast cancer is a triple negative breast cancer.

In some embodiments, the ovarian cancer is an epithelial ovarian cancer.

In some embodiments, the anti-TROP-2 antibody-drug conjugate and the additional therapeutic agent are present in a unit formulation.

In some embodiments, the anti-TROP-2 antibody-drug conjugate and the additional therapeutic agent are present in the same formulation unit.

In some embodiments, the anti-TROP-2 antibody-drug conjugate and the additional therapeutic agents are present in separate formulation units.

In some embodiments, the anti-TROP-2 antibody-drug conjugate and the additional therapeutic agents are administered simultaneously.

In some embodiments, the anti-TROP-2 antibody-drug conjugate and the additional therapeutic agent are administered separately.

In some embodiments, the anti-TROP-2 antibody-drug conjugate and the additional therapeutic agent are administered sequentially.

In some embodiments, the anti-TROP-2 antibody-drug conjugate is administered first, and the additional therapeutic agent is administered subsequently.

In some embodiments, the cycle of administration of the antibody-drug conjugate is 14 to 42 days. In some embodiments, the cycle of administration of the antibody-drug conjugate is 14 days, 21 days, 28 days or 42 days. In some embodiments, the cycle of administration of the antibody-drug conjugate is 14 days. In some embodiments, the cycle of administration of the antibody-drug conjugate is 21 days.

In some embodiments, the cycle of administration of the additional therapeutic agent is 14 to 42 days, such as 14 days, 21 days, 28 days, or 42 days.

In some embodiments, the cycle of administration of the anti-PD-L1 antibody or antigen-binding fragment thereof is 14 to 42 days. In some embodiments, the cycle of administration of the anti-PD-L1 antibody or antigen-binding fragment thereof is 14 days, 21 days, 28 days, or 42 days. In some embodiments, the cycle of administration of the anti-PD-L1 antibody or antigen-binding fragment thereof is 14 days; in some embodiments, the cycle of administration of the anti-PD-L1 antibody or antigen-binding fragment thereof is 21 days.

In some embodiments, the cycle of administration of the platinum drug (e.g., Carboplatin or Cisplatin) is 21 to 42 days; in some embodiments, the cycle of administration of the platinum drug (e.g., Carboplatin or Cisplatin) is 21 days, 28 days, or 42 days; in some embodiments, the cycle of administration of the platinum drug (e.g., Carboplatin or Cisplatin) is 21 days; in some embodiments, the cycle of administration of the platinum drug (e.g., Carboplatin or Cisplatin) is 28 days; in some embodiments, the cycle of administration of the platinum drug (e.g., Carboplatin or Cisplatin) is 42 days.

In some embodiments, the antibody-drug conjugate is administered at a dose selected from the group consisting of 1 mg/kg to 10 mg/kg, preferably 1 mg/kg, 2 mg/kg, 3 mg/kg, 4 mg/kg, 5 mg/kg, 6 mg/kg, 7mg/kg, 8mg/kg, 9mg/kg, and 10mg/kg per time. In some embodiments, the antibody-drug conjugate is administered at a dose of 4 mg/kg or 5 mg/kg per time.

In some embodiments, the anti-PD-L1 antibody or antigen-binding fragment thereof is administered at a dose selected from the group consisting of 900 mg to 1500 mg, preferably 900 mg, 1000 mg, 1100 mg, 1200 mg, 1300 mg, 1400 mg, and 1500 mg per time.

In some embodiments, the anti-PD-L1 antibody or antigen-binding fragment thereof is administered at a dose of 900 mg or 1200 mg per time.

In some embodiments, the non-small cell lung cancer is an advanced non-small cell lung cancer that has not received a systemic therapy or has received at most a first-line chemotherapy, and the anti-PD-L1 antibody or antigen-binding fragment thereof is administered at a dose of 900 mg or 1200 mg per time.

In some embodiments, the antibody-drug conjugate is administered at a dose of 4 mg/kg or 5 mg/kg per time, and the non-small cell lung cancer is an advanced non-small cell lung cancer that has not received a systematic therapy or has received at most a first-line chemotherapy, and the anti-PD-L1 antibody or antigen-binding fragment thereof is administered per time at a dose of 900 mg or 1200 mg.

In some embodiments, the breast cancer is a triple negative breast cancer, and the anti-PD-L1 antibody or antigen-binding fragment thereof is administered at a dose of 900 mg or 1200 mg per time.

In some embodiments, the antibody-drug conjugate is administered at a dose of 4 mg/kg or 5 mg/kg per time, the breast cancer is a triple-negative breast cancer, and the anti-PD-L1 antibody or antigen-binding fragment thereof is administered per time at a dose of 900mg or 1200mg.

In some embodiments, the anti-TROP-2 antibody-drug conjugate and the additional therapeutic agent comprising the anti-PD-L1 antibody or antigen-binding fragment thereof and the chemotherapeutic agent are used in the manufacture of a medicament for treating a non-small cell lung cancer, a triple-negative breast cancer, or an ovarian cancer, wherein the chemotherapeutic agent is one or more selected from the group consisting of Carboplatin or Cisplatin.

In some embodiments, Carboplatin is administered at a dose, calculated as area under curve (AUC), of 1 mg/ml/min to 10 mg/ml/min, preferably 1 mg/ml/min, 2 mg/ml/min, 2.5 mg/ml/min, 3 mg/ml/min, 3.75 mg/ml/min, 4 mg/ml/min, 5 mg/ml/min, 6 mg/ml/min, 7 mg/ml/min, 8 mg/ml/min per time. In some embodiments, Carboplatin is administered at a dose of 5 mg/ml/min, or 3.75 mg/ml/min, or 2.5 mg/ml/min per time.

In some embodiments, Cisplatin is administered at a dose of 50 mg/m² to 150 mg/m², preferably from 50 mg/m², 75 mg/m², 100 mg/m², 120 mg/m² and 150 mg/m² per time.

In some embodiments, the antibody-drug conjugate and the anti-PD-L1 antibody or antigen-binding fragment thereof are administered per time at doses as described above.

In some embodiments, the subject is a patient with non-small cell lung cancer who previously neither has received an immune checkpoint inhibitor therapy, nor has received or has received at most a first-line systemic chemotherapy regimen.

In some embodiments, the subject is a patient with non-small cell lung cancer who previously has received an adjuvant, neoadjuvant or radical chemoradiotherapy, and the cancer progresses during the therapy or within 6 months after the end of the therapy.

In some embodiments, the subject is a patient with non-small cell lung cancer who has received but been failed by an EGFR kinase inhibitor therapy.

In some embodiments, the subject previously has received but been failed by a first- or second-generation EGFR tyrosine kinase inhibitor (TKI) therapy, and the T790M mutation in exon 20 of EGFR is negative.

In some embodiments, the subject previously has received but been failed by a third-generation EGFR tyrosine kinase inhibitor (TKI) therapy.

In some embodiments, the subject previously neither has received a systemic anti-tumor therapy for a locally advanced or metastatic non-small cell lung cancer, nor has received an immune checkpoint inhibitor therapy.

In some embodiments, the subject is a patient with an unresectable locally advanced, recurrent, or metastatic triple-negative breast cancer who previously has not received a systemic therapy.

Another aspect of the present invention also provides a method for treating a cancer in a subject, which comprises a step of administering to the subject a therapeutically effective amount of the anti-TROP-2 antibody-drug conjugate and additional therapeutic agent as described above.

In some embodiments, the additional therapeutic agent is one or more selected from the group consisting of the anti-PD-L1 antibody or antigen-binding fragment thereof and the chemotherapeutic drug as described above.

In some embodiments, the TROP-2-targeting antibody-drug conjugate comprises a camptothecin drug and an anti-TROP-2 antibody or antigen-binding fragment thereof, and the anti-TROP-2 antibody or antigen-binding fragment thereof comprises a heavy chain variable region and a light chain variable region, wherein the heavy chain variable region comprises a HCDR1 as set forth in SEQ ID No.: 1, a HCDR2 as set forth in SEQ ID No.: 2 and a HCDR3 as set forth in SEQ ID No.: 3, and the light chain variable region comprises a LCDR1 as set forth in SEQ ID No.: 4, a LCDR2 as set forth in SEQ ID No.: 5 and a LCDR3 as set forth in SEQ ID No.: 6.

In some embodiments, the anti-PD-L1 antibody or antigen-binding fragment thereof is one or more selected from the group consisting of Durvalumab, Atezolizumab, and Envafolimab.

In some embodiments, the anti-PD-L1 antibody or antigen-binding fragment thereof comprises a heavy chain variable region and a light chain variable region, wherein the heavy chain variable region comprises a HCDR1 as set forth in SEQ ID No.: 7, a HCDR2 as set forth in SEQ ID No.: 8 and a HCDR3 as set forth in SEQ ID No.: 9, and the light chain variable region comprises a LCDR1 as set forth in SEQ ID No.: 10, a LCDR2 as set forth in SEQ ID No.: 11 and a LCDR3 as set forth in SEQ ID No.: 12.

In some embodiments, the chemotherapeutic drug is selected from platinum drugs.

In some embodiments, the platinum drug is selected from the group consisting of Cisplatin, Carboplatin, Sulfatodiaminocyctohexane Platin, Nedaplatin, Oxaliplatin, Lobaplatin, Satraplatin, Miboplatin, Enloplatin, Iproplatin, and Dicycloplatin.

In some embodiments, the anti-TROP-2 antibody comprises a heavy chain as set forth in SEQ ID No.: 13 and a light chain as set forth in SEQ ID No.: 14.

In some embodiments, the anti-PD-L1 antibody or antigen-binding fragment thereof comprises a heavy chain variable region as set forth in SEQ ID No.: 15 and a light chain variable region as set forth in SEQ ID No.: 16.

In some embodiments, the anti-PD-L1 antibody or antigen-binding fragment thereof comprises a mutation in the amino acid sequence of the anti-PD-L1 antibody of the present invention. The variant amino acid sequence is identical to a reference sequence except for having a substitution, deletion and/or addition of one, two, three, four or five amino acids.

In some embodiments, the substitution, deletion and/or addition is located in a CDR. In some embodiments, the substitution, deletion, and/or addition is located in a framework region.

In some embodiments, the substitution of one, two, three, four or five amino acids is a conservative substitution.

In some embodiments, the anti-PD-L1 antibody or antigen-binding fragment thereof has a V_{L} domain with a sequence homology of at least 95%, 90%, 85%, 80%, 75% or 50% to one of the V_{L} domains of the anti-PD-L1 antibody of the present invention, and exhibits specific binding to PD-L 1.

In some embodiments, the anti-PD-L1 antibody or antigen-binding fragment thereof has a V_{H} domain with a sequence homology of at least 95%, 90%, 85%, 80%, 75% or 50% to one of the V_{H} domains of the anti-PD-L1 antibody of the present invention, and exhibits specific binding to PD-L 1.

In some embodiments, the anti-PD-L1 antibody or antigen-binding fragment thereof has a V_{L} domain with a substitution, deletion and/or addition of up to 1, 2, 3, 4, 5 or more amino acids as compared to one of the V_{L} domains of the anti-PD-L1 antibody of the present invention, and exhibits specific binding to PD-L 1.

In some embodiments, the anti-PD-L1 monoclonal antibody or antigen-binding fragment thereof has a V_{H} domain with a substitution, deletion and/or addition of up to 1, 2, 3, 4, 5 or more amino acids as compared to one of the V_{H} domains of the anti-PD-L1 antibody of the present invention, and exhibits specific binding to PD-L1.

In some embodiments, the anti-PD-L1 antibody or antigen-binding fragment thereof has a V_{L} domain with a substitution, deletion and/or addition of up to 1, 2, 3, 4, 5 or more amino acids as compared to one of the V_{L} domains of the anti-PD-L1 antibody of the present invention, and a V_{H} domain with a substitution, deletion and/or addition of up to 1, 2, 3, 4, 5 or more amino acids as compared to one of the V_{H} domains of the anti-PD-L1 antibody of the present invention, and exhibits specific binding to PD-L 1.

Generally, the variant of the anti-PD-L1 monoclonal antibody and antigen-binding fragment thereof disclosed in the present invention has an amino acid sequence with an amino acid sequence identity of at least 75%, more preferably at least 80%, more preferably at least 85%, more preferably at least 90%, most preferably at least 95%, 98% or 99% as compared to an amino acid sequence of a reference antibody or antigen-binding fragment (e.g., a heavy chain, light chain, V_{H}, V_{L}, or humanized sequence). The terminology "identity" or "homology" in the present invention is defined as percentage of the amino acid residues in a candidate sequence that are identical to a reference sequence, if necessary, after aligning the sequences and introducing gaps to achieve maximum percent sequence identity, and not considering any conservative substitutions as part of sequence identity. N-terminus, C-terminus, or internal extensions, deletions, or insertions of antibody sequences shall not be interpreted as affecting sequence identity or homology.

In some embodiments, the anti-PD-L1 monoclonal antibody is a human antibody. In other embodiments, the anti-PD-L1 monoclonal antibody is a humanized antibody.

In some embodiments, the antibody-drug conjugate has the following chemical structure: 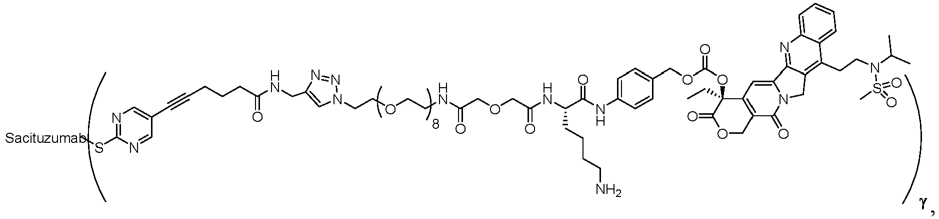 wherein γ represents the average number of drug-linker units conjugated to each antibody molecule in the antibody-drug conjugate, and the average number is in the range of 6 to 10. In some embodiments, the average number of γ is between 6 and 9. In some embodiments, the average number of γ is between 6 and 8, for example about 6.1, 6.2, 6.3, 6.4, 6.5, 6.6, 6.7, 6.8, 6.9, 7, 7.1, 7.2, 7.3, 7.4, 7.5, 7.6, 7.7, 7.8 or 7.9.

In some embodiments, the cancer is a solid tumor or a non-solid tumor; for example, selected from the group consisting of esophageal cancer (e.g., esophageal adenocarcinoma and squamous cell esophageal carcinoma), brain tumor, lung cancer (e.g., small cell lung cancer and non-small cell lung cancer), squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor (e.g., neuroglioma, glioblastoma multiforme, glioma, or sarcoma), prostate cancer, and thyroid cancer.

In some embodiments, the cancer is selected from the group consisting of lung cancer, breast cancer, and epithelial ovarian cancer.

In some embodiments, the cancer is non-small cell lung cancer (NSCLC) or triple negative breast cancer (TNBC).

In some embodiments, the non-small cell lung cancer is selected from EGFR wild-type or mutated non-small cell lung cancer.

In some embodiments, the non-small cell lung cancer is an advanced/metastatic non-small cell lung cancer.

In some embodiments, the non-small cell lung cancer is an advanced non-small cell lung cancer that has not been treated with a systemic therapy or has received at most a first-line chemotherapy.

In some embodiments, the non-small cell lung cancer is an ALK rearrangements-negative non-small cell lung cancer.

In some embodiments, the EGFR mutation is single mutation or compound mutation.

In some embodiments, the breast cancer is selected from the group consisting of triple negative breast cancer, HR+/HER2- breast cancer, HR-/HER2+ breast cancer, HR+/HER2+ breast cancer, and BRCA+ breast cancer.

In some embodiments, the breast cancer is a triple negative breast cancer.

In some embodiments, the ovarian cancer is an epithelial ovarian cancer.

In some embodiments, the cancer is a non-small cell lung cancer as described above.

In some embodiments, the administration frequency of the anti-TROP-2 antibody-drug conjugate combined with the anti-PD-L1 antibody or antigen-binding fragment thereof and/or chemotherapeutic agent for treating the non-small cell lung cancer as described above is once every 2 weeks or once every 3 weeks.

In some embodiments, the cancer is the breast cancer as described above, preferably a triple negative breast cancer.

In some embodiments, the administration frequency of the anti-TROP-2 antibody-drug conjugate combined with the anti-PD-L1 antibody or antigen-binding fragment thereof and/or chemotherapeutic agent for treating the breast cancer as described above is once every 2 weeks or once every 3 weeks.

In some embodiments, the cycle of administration of the antibody-drug conjugate is 14 to 42 days. In some embodiments, the cycle of administration of the antibody-drug conjugate is 14 days, 21 days, 28 days or 42 days. In some embodiments, the cycle of administration of the antibody-drug conjugate is 14 days. In some embodiments, the cycle of administration of the antibody-drug conjugate is 21 days.

In some embodiments, the cycle of administration of the additional therapeutic agent is 14 to 42 days, such as 14 days, 21 days, 28 days, or 42 days.

In some embodiments, the cycle of administration of the anti-PD-L1 antibody or antigen-binding fragment thereof is 14 to 42 days. In some embodiments, the cycle of administration of the anti-PD-L1 antibody or antigen-binding fragment thereof is 14 days, 21 days, 28 days, or 42 days. In some embodiments, the cycle of administration of the anti-PD-L1 antibody or antigen-binding fragment thereof is 14 days; in some embodiments, the cycle of administration of the anti-PD-L1 antibody or antigen-binding fragment thereof is 21 days.

In some embodiments, the cycle of administration of the platinum drug (e.g., Carboplatin or Cisplatin) is 21 to 42 days; in some embodiments, the cycle of administration of the platinum drug (e.g., Carboplatin or Cisplatin) is 21 days, 28 days, or 42 days; in some embodiments, the cycle of administration of the platinum drug (e.g., Carboplatin or Cisplatin) is 21 days; in some embodiments, the cycle of administration of the platinum drug (e.g., Carboplatin or Cisplatin) is 28 days; in some embodiments, the cycle of administration of the platinum drug (e.g., Carboplatin or Cisplatin) is 42 days.

In some embodiments, the anti-TROP-2 antibody-drug conjugate and the additional therapeutic agent are administered simultaneously.

In some embodiments, the anti-TROP-2 antibody-drug conjugate and the additional therapeutic agent are administered separately.

In some embodiments, the anti-TROP-2 antibody-drug conjugate and the additional therapeutic agent are administered sequentially.

In some embodiments, the anti-TROP-2 antibody-drug conjugate is administered first, and the additional therapeutic agent is administered subsequently.

In some embodiments, the antibody-drug conjugate is administered at a dose of 1 mg/kg to 10 mg/kg, preferably 1 mg/kg, 2 mg/kg, 3 mg/kg, 4 mg/kg, 5 mg/kg, 6 mg/kg, 7mg/kg, 8mg/kg, 9mg/kg or 10mg/kg per time. In some embodiments, the antibody-drug conjugate is administered at a dose of 4 mg/kg or 5 mg/kg per time.

In some embodiments, the anti-PD-L1 antibody or antigen-binding fragment thereof is administered at a dose of 900 mg to 1500 mg, preferably 900 mg, 1000 mg, 1100 mg, 1200 mg, 1300 mg, 1400 mg, 1500 mg per time.

In some embodiments, the anti-PD-L1 antibody or antigen-binding fragment thereof is administered at a dose of 900 mg or 1200 mg per time.

In some embodiments, the non-small cell lung cancer is an advanced non-small cell lung cancer that has not received a systemic therapy or has received at most a first-line chemotherapy, and the anti-PD-L1 antibody or antigen-binding fragment thereof is administered at a dose of 900 mg or 1200 mg per time.

In some embodiments, the antibody-drug conjugate is administered at a dose of 4 mg/kg or 5 mg/kg per time, and the non-small cell lung cancer is an advanced non-small cell lung cancer that has not received a systematic therapy or has received at most a first-line chemotherapy, and the anti-PD-L1 antibody or antigen-binding fragment thereof is administered per time at a dose of 900 mg or 1200 mg.

In some embodiments, the breast cancer is a triple negative breast cancer, and the anti-PD-L1 antibody or antigen-binding fragment thereof is administered at a dose of 900 mg or 1200 mg per time.

In some embodiments, the antibody-drug conjugate is administered at a dose of 4 mg/kg or 5 mg/kg per time, the breast cancer is a triple-negative breast cancer, and the anti-PD-L1 antibody or antigen-binding fragment thereof is administered per time at a dose of 900mg or 1200mg.

In some embodiments, the method is used for treating a non-small cell lung cancer, a triple negative breast cancer, or an ovarian cancer, which comprises a step of administering a subject therapeutically effective amounts of the anti-TROP-2 antibody-drug conjugate and an additional therapeutic agent, wherein the additional therapeutic agent comprises an anti-PD-L1 antibody or antigen-binding fragment thereof and a chemotherapeutic drug, and the chemotherapeutic drug is one or more selected from the group consisting of Carboplatin or Cisplatin.

In some embodiments, the Carboplatin is administered at a dose, calculated as area under curve (AUC), of 1 mg/ml/min to 10 mg/ml/min, preferably 1 mg/ml/min, 2 mg/ml/min, 2.5 mg /ml/min, 3 mg/ml/min, 3.75 mg/ml/min, 4 mg/ml/min, 5 mg/ml/min, 6 mg/ml/min, 7 mg/ml/min, 8 mg/ml/min per time. In some embodiments, the Carboplatin is administered at a dose of 5 mg/ml/min, or 3.75 mg/ml/min, or 2.5 mg/ml/min per time.

In some embodiments, the Cisplatin is administered per time at a dose of 50 mg/m² to 150 mg/m², preferably from 50 mg/m², 75 mg/m², 100 mg/m², 120 mg/m² and 150 mg/m².

In some embodiments, the antibody-drug conjugate and the anti-PD-L1 antibody or antigen-binding fragment thereof are administered per time at doses as described above.

In some embodiments, the subject is a patient with non-small cell lung cancer who previously neither has received an immune checkpoint inhibitor therapy, nor has received or has received at most a first-line systemic chemotherapy regimen.

In some embodiments, the subject is a patient with non-small cell lung cancer who previously has received an adjuvant, neoadjuvant or radical chemoradiotherapy, and the cancer progresses during the therapy or within 6 months after the end of the therapy.

In some embodiments, the subject is a patient with non-small cell lung cancer who has received but been failed by an EGFR kinase inhibitor therapy.

In some embodiments, the subject previously has received but been failed by a first- or second-generation EGFR tyrosine kinase inhibitor (TKI) therapy, and the T790M mutation in exon 20 of EGFR is negative.

In some embodiments, the subject previously has received but been failed by a third-generation EGFR tyrosine kinase inhibitor (TKI) therapy.

In some embodiments, the subject previously neither has received a systemic anti-tumor therapy for a locally advanced or metastatic non-small cell lung cancer, nor has received an immune checkpoint inhibitor therapy.

In some embodiments, the subject is a patient with an unresectable locally advanced, recurrent, or metastatic triple-negative breast cancer who previously has not received a systemic therapy.

Another aspect of the present invention also provides the anti-TROP-2 antibody-drug conjugate and the additional therapeutic agent as described above for treating a cancer in a subject, wherein the additional therapeutic agent comprises one or more selected from the group consisting of the anti-PD-L1 antibody or antigen-binding fragments thereof and the chemotherapeutic agent.

Another aspect of the present invention also provides one or more kits, which comprise the combination of the anti-TROP-2 antibody-drug conjugate and the additional therapeutic agent as described above.

In some embodiments, the kit is used for detection before and/or during treatment.

In some embodiments, the kit comprises the anti-TROP-2 antibody-drug conjugate as described above in one compartment, the anti-PD-L1 antibody or antigen-binding fragment thereof as described above in another compartment, and Carboplatin in yet another compartment.

In some embodiments, the kit comprises the anti-TROP-2 antibody-drug conjugate as described above in one compartment, the anti-PD-L1 antibody or antigen-binding fragment thereof as described above in another compartment, and Cisplatin in yet another compartment.

In some embodiments, the kit further comprises an instruction for administering the anti-TROP-2 antibody-drug conjugate, the anti-PD-L1 antibody or antigen-binding fragment thereof, Carboplatin or Cisplatin.

In some embodiments, the kit is used for treating a cancer as described above.

Another aspect of the present invention also provides a pharmaceutical composition, which comprises the anti-TROP-2 antibody-drug conjugate as described above, and comprises one or more of the anti-PD-L1 antibody or antigen-binding fragment thereof and chemotherapeutic agent as described above.

In some embodiments, the pharmaceutical composition further comprises one or more pharmaceutically acceptable excipients, diluents or carriers.

Another aspect of the present invention also provides use of the anti-TROP-2 antibody-drug conjugate as describe above and the one or more of the anti-PD-L1 antibody or antigen-binding fragment thereof and the chemotherapeutic agent as described above in the manufacture of a medicament for treating a tumor.

Another aspect of the present invention also provides use of a combination of the anti-TROP-2 antibody-drug conjugate as described above and the anti-PD-L1 antibody or antigen-binding fragment thereof in the manufacture of a medicament for treating a tumor.

Another aspect of the present invention also provides use of a combination of the anti-TROP-2 antibody-drug conjugate as described above, the anti-PD-L1 antibody or antigen-binding fragment thereof, and Carboplatin/Cisplatin in the manufacture of a medicament for treating a tumor.

The present invention provides a method for treating a cancer as described above by using a combination of the anti-TROP-2 antibody-drug conjugate as described above and the additional therapeutic agent (e.g., the anti-PD-L1 antibody or antigen-binding fragment thereof, chemotherapeutic agent).

The present invention provides use of a therapeutic combination for treating a cancer as described above, wherein the therapeutic combination comprises the anti-TROP-2 antibody-drug conjugate as described above, and the additional therapeutic agent (e.g., the anti-PD-L1 antibody or antigen-binding fragment thereof, chemotherapeutic agent).

The present invention also provides a therapeutic combination for treating a cancer as described above, comprising administering a subject in need thereof:
(a) the anti-TROP-2 antibody-drug conjugate as described above;
(b) the additional therapeutic agent as described above (e.g., the anti-PD-L1 antibody or antigen-binding fragment thereof, chemotherapeutic agent).

In some embodiments, the method reduces tumor size by at least about 10%, such as about 10%, 20%, 30%, or more.

In some embodiments, the method reduces tumor size by about 10%, 15%, 20%, 25%, 30%, 35%, 40%, 45%, 50%, 55%, 60%, 65%, 70%, 75%, 80%, 85%, 90%, 95% or 100%.

In some embodiments, the method inhibits the growth of a tumor and stabilizes a disease.

### Definition of Terms

Certain technical and scientific terms are specifically defined below. Unless otherwise specified, all other technical and scientific terms used herein have the meaning commonly understood by one of ordinary skill in the art to which this disclosure relates.

The term "antibody" refers to an immunoglobulin molecule that recognizes and specifically binds to a target, such as a protein, polypeptide, peptide, carbohydrate compound, polynucleotide, lipid, or combination thereof, through at least one antigen recognition site within the variable region of the immunoglobulin molecule. As used herein, the term "antibody" encompasses intact polyclonal antibody, intact monoclonal antibody, chimeric antibody, humanized antibody, human antibody, antibody-containing fusion protein, and any other modified immunoglobulin molecule, so long as the antibody exhibits the desired biological activity. Antibodies can belong to any of the five main classes of immunoglobulins: IgA, IgD, IgE, IgG, and IgM, or their subclasses (isotypes) (e.g., IgG1, IgG2, IgG3, IgG4, IgA1, and IgA2), based on the properties of their heavy chain constant domains, known as α, δ, ε, γ, and µ, respectively. Different classes of immunoglobulins have different and well-understood subunit structures and three-dimensional configurations. Antibodies can be naked or conjugated to other molecules, such as toxins, radioisotopes, and the like.

The term "antibody fragment" refers to a portion of an intact antibody. "Antigen-binding fragment" refers to a portion of an intact antibody that binds to an antigen. The antigen-binding fragment may comprise an antigen-determining variable region of the intact antibody. Examples of antibody fragments comprise, but are not limited to, Fab, Fab', F(ab')₂ and Fv fragment, linear antibody and single chain antibody.

The term "anti-TROP-2 antibody" or "TROP-2-targeting antibody" refers to an antibody capable of binding TROP-2 with sufficient affinity such that the antibody can be used as a diagnostic and/or therapeutic agent targeting TROP-2 (e.g. RS7 antibody).

The term "anti-PD-L1 antibody" or "PD-L1-targeting antibody" refers to an antibody that is capable of specifically binding PD-L1 with sufficient affinity such that the antibody can be used as a therapeutic agent targeting PD-L1. Anti-PD-L1 antibody binds to an unrelated non-PD-L1 protein to an extent that is less than about 10% of the extent of the antibody binding to PD-1, for example, as measured by radioimmunoassay (RIA). In certain embodiments, the antibody that binds to PD-L1 has a dissociation constant (Kd) of ≤1 µM, ≤100 nM, ≤10 nM, ≤1 nM, or ≤0.1 nM. In certain embodiments, an antibody or antigen-binding fragment thereof that binds to PD-1 comprises three heavy chain CDR-1, CDR-2 and CDR-3 sequences as set forth in SEQ ID No.: 7, SEQ ID No.: 8 and SEQ ID No.: 9, respectively, and three light chain CDR-1, CDR-2 and CDR-3 sequences as set forth in SEQ ID No.: 10, SEQ ID No.: 11 and SEQ ID No.: 12, respectively. In certain embodiments, the antibody or antigen-binding fragment thereof that binds to PD-1 further comprises a heavy chain variable region sequence as set forth in SEQ ID No.: 18 and a light chain variable region sequence as set forth in SEQ ID No.: 19.

The term "x-line treatment" or "x-line therapy" refers to a therapeutic regimen that may comprise (but is not limited to) surgery, radiotherapy, chemotherapy, differentiation therapy, biological therapy, immunotherapy, or administration of one or more anticancer agents (e.g., cytotoxic agents, antiproliferative compounds, and/or angiogenesis inhibitors).

The terms "first-line treatment," "first-line therapy," and "front-line therapy" refer to the preferred standard initial therapy for a particular condition (e.g., a cancer of given type and stage), which is different from a "second-line" therapy that is tried when the first-line therapy doesn't work appropriately. A "third-line" therapy is tried when first- and second-line therapies don't work appropriately.

For example, the combination of anti-TROP-2 antibody-drug conjugate and anti-PD-L1 antibody or antigen-binding fragment thereof as provided herein can be administered as a first-line therapy, a second-line therapy, or a third-line therapy.

The combination of anti-TROP-2 antibody-drug conjugate and anti-PD-L1 antibody or antigen-binding fragment thereof as provided herein can be administered as a first-line therapy to a patient who previously has received 0, 1, 2, 3, 4, 5, 6 or more lines of therapies, but has not yet received a combination therapy of anti-TROP-2 antibody-drug conjugate and anti-PD-L1 antibody or antigen-binding fragment thereof as provided herein.

The combination of anti-TROP-2 antibody-drug conjugate and anti-PD-L1 antibody or antigen-binding fragment thereof as provided herein can be administered as a first-line therapy to a patient who previously has received at least 1 line, at least 2 lines, or at least 3 lines of therapies, but has not yet received a combination therapy of anti-TROP-2 antibody-drug conjugate and anti-PD-L1 antibody or antigen-binding fragment thereof as provided herein.

In some embodiments, the combination of anti-TROP-2 antibody-drug conjugate and anti-PD-L1 antibody or antigen-binding fragment thereof as provided herein can be administered as a first-line therapy to a patient who has received no more than 1 line, no more than 2 lines, no more than 3 lines, no more than 4 lines, no more than 5 lines, no more than 6 lines of therapies.

In certain embodiments, the combination of anti-TROP-2 antibody-drug conjugate and anti-PD-L1 antibody or antigen-binding fragment thereof as provided herein can be administered as an adjuvant therapy, a neoadjuvant therapy, or a maintenance therapy.

The term "adjuvant therapy" refers to a systemic therapy administered after surgery. Broadly speaking, adjuvant therapy is a therapy administered in addition to a primary therapy to kill any cancer cells that may have spread, even if the spread cannot be detected by radiographic imaging or laboratory test.

The term "neoadjuvant therapy" refers to a systemic therapy administered before surgery.

The term "maintenance therapy" refers to a therapy that helps prevent a cancer from reoccurring after it disappears after initial therapy.

The term "RS7 antibody" refers to the commercial antibody Sacituzumab that targets TROP-2, and is an anti-TROP-2 antibody comprising the three heavy chain CDRs sequences, CDR-1, CDR-2 and CDR-3, as set forth in SEQ ID No.: 1, SEQ ID No.: 2 and SEQ ID No.: 3, respectively, and the three light chain CDRs sequences, CDR-1, CDR-2 and CDR-3, as set forth in SEQ ID No.:4, SEQ ID No.:5 and SEQ ID No.:6, respectively (see, U.S. Patent No. 7,517,964). The anti-TROP-2 antibody RS7 that can be used in the present disclosure can also be screened and obtained by the methods of vector design and construction, and construction of antibody library for displaying antibodies as disclosed in CN103476941A, or can be screened and obtained by G-MAB^{®} library of Sorrento Therapeutics, Inc.

The term "linker" refers to any chemical moiety capable of linking a compound (usually a drug, such as camptothecin and derivatives thereof) to a cell-binding agent (e.g., an anti-TROP-2 antibodies or fragment thereof) in a stable covalent manner. The linker tends to be resistant or substantially resistant to, for example, disulfide bond cleavage under conditions that allow the compound or antibody to remain active.

The terms "cancer" and "carcinoma" refer to or describe a pathological condition in a mammal in which a population of cells is characterized by unregulated cell growth. Examples of cancer comprise ovarian cancer, fallopian tube cancer, and peritoneal cancer. Another example of cancer is endometrial cancer. The cancer may be a cancer that expresses TROP-2 ("TROP-2-expressing cancer").

The terms "cancer cell," "tumor cell" and grammatical equivalents thereof refer to a total population of cells derived from a tumor or precancerous lesion, which comprises non-tumorigenic cells comprising a large number of populations of tumor cells, and tumorigenic stem cells (cancer stem cells). As used herein, the term "tumor cell" when referring only to a tumor cell that lacks the ability to renew and differentiate will be modified by the term "non-tumorigenic" to distinguish the tumor cell from a cancer stem cell.

"Advanced" cancer refers to a cancer that has spread beyond the original site or organ through local invasion or metastasis. The term "advanced" cancer comprises both locally advanced disease and metastatic disease.

"Metastatic" cancer refers to a cancer that spreads from one part of the body to another.

"Refractory" cancer refers to a cancer that progresses even when a patient bearing the cancer is administered with an anti-tumor therapy, such as chemotherapy.

"Recurrent" cancer refers to a cancer that grows again at the original site or at a remote site after the response to initial therapy.

The term "subject" refers to any animal (e.g., mammal) receiving a particular therapy, and includes (but is not limited to) non-human primate, rodent, and the like. Typically, the terms "subject" and "patient" are used interchangeably herein when referring to a human individual. The mammal may be one or more species selected from the group consisting of human, bovine (e.g., cow), porcine (e.g., pig), ovine (e.g., sheep), caprine (e.g., goat), equid (e.g., horse), canine (e.g., domestic dog), feline (e.g., domestic cat), lagomorph (e.g., rabbit), rodent (e.g., rat or mouse), etc. In certain embodiments, the subject is a human.

"Relapsing" patient refers to a patient who has recurrent conditions or symptoms of cancer after remission. Optionally, the patient relapses after adjuvant or neoadjuvant therapy.

Administration "in combination with one or more additional therapeutic agents" comprises simultaneous (concurrent) or sequential administration in any order.

Combination therapy may provide "synergistic effect" and be demonstrated "synergism," that is, when the active ingredients are used together, they achieve an effect that exceeds the sum of the effects caused by using each of compounds individually. Synergistic effect is achieved when: (1) the active ingredients are co-formulated and administered or delivered simultaneously in a combined unit dose formulation; (2) the active ingredients are delivered sequentially, alternately, or in parallel in separate formulations; or (3) the active ingredients are each administered by some other regimen. When delivered as an alternating regimen, a synergistic effect can be obtained if the compounds are administered or delivered sequentially, for example, by separate injections with separate syringes. A synergistic combination produces an effect that exceeds the sum of the effects of individual components of the combination.

The therapeutic agent and composition provided by the disclosure may be administered by any suitable enteral or parenteral route of administration. The term "enteral route" administration refers to administration through any part of the gastrointestinal tract. Examples of enteral routes comprise oral, mucosal, buccal and rectal routes, or intragastric route. "Parenteral" administration refers to administration by a route other than the enteral route. Examples of parenteral routes of administration comprise intravenous, intramuscular, intradermal, intraperitoneal, intratumoral, intravesical, intraarterial, intrathecal, intrasaccular, intraorbital, intracardiac, transtracheal, intraarticular, infracapsule, subarachnoid, intraspinal, epidural and intrasternal, subcutaneous or topical administration. The therapeutic agent and composition of the present disclosure may be administered by any suitable method, such as by injection, infusion, implantable infusion pump, and osmotic pump. Appropriate routes and methods of administration may vary depending on many factors, such as the specific therapeutic agent used, the desired rate of absorption, the specific formulation or dosage form used, the type or severity of the disease being treated, the specific site of action, and the patient's conditions, and can be easily selected by those skilled in the art.

An "effective amount" of the antibody, antibody-drug conjugate, or other drug disclosed herein refers to an amount sufficient to achieve the specifically stated purpose. An "effective amount" can be determined empirically and in a routine manner for the stated purpose.

The term "therapeutically effective amount" refers to an amount of the antibody, immunoconjugate or other drug that is effective to "treat" a disease or condition in an individual or mammal. In the case of cancer, a therapeutically effective amount of drug can reduce the number of cancer cells; reduce tumor size or burden; inhibit (i.e., slow down to a certain extent, and, in one embodiment, stop) the infiltration of cancer cells into surrounding organs; inhibit (i.e., slow down to a certain extent, and, in one embodiment, stop) tumor metastasis; inhibit tumor growth to a certain extent; alleviate one or more cancer-related symptoms to a certain extent; and/or induce a favorable response, such as prolonged progression-free survival (PFS), disease-free survival (DFS) or overall survival (OS), complete remission (CR), partial remission (PR), or in some cases, stable disease (SD), delayed progressive disease (PD), prolonged time to progression (TTP), or any combination thereof. See the definition of "treatment" herein. To the extent that the drug prevents the growth of cancer cells and/or kills existing cancer cells, the drug may be a cytostatic and/or cytotoxic drug.

PFS, DFS, and OS can be measured according to the standards set by the National Cancer Institute and the U.S. Food and Drug Administration for new drug approvals. See Johnson et al., J. Clin. Oncol. 21(7):1404-1411 (2003).

"Progression-free survival" (PFS) refers to the time from registration to disease progression or death. PFS is generally measured using the Kaplan-Meier method and Response Evaluation Criteria in Solid Tumors (RECIST), 1.1 standards. Generally speaking, progression-free survival refers to a state in which a patient remains alive without deterioration of cancer.

"Time to tumor progression" (TTP) is defined as the time from registration to disease progression. TTP is generally measured using the RECIST 1.1 standard.

"Complete remission" or "CR" indicates that all lesions of tumor or cancer have disappeared in response to treatment. This does not always mean the cancer is cured.

"Partial remission" or "PR" refers to a decrease in the size or volume of one or more tumors or lesions, or the extent of cancer in the body, in response to treatment.

"Stable disease" refers to no progression or recurrence of disease. In terms of stable disease, neither sufficient tumor shrinkage to qualify for partial response, nor sufficient increase to qualify for progressive disease is achieved.

"Progressive disease" refers to the appearance of one or more new lesions or tumors and/or significant progression of existing non-target lesions. Progressive disease may also refer to more than 20% tumor growth from the start of treatment due to mass gain or tumor spread.

"Disease-free survival" (DFS) refers to a length of time that a patient remains disease-free during and after treatment.

"Overall survival" (OS) refers to a time from patient registration to death or the last known date of survival at censorship. OS comprises the extension in life expectancy compared to untreated individuals or patients. Overall survival refers to a state in which a patient remains alive for a specified period of time, such as one year, five years, etc. from the time of diagnosis or treatment. Within the patient population, overall survival is measured as median overall survival (mOS).

"Prolonged survival" or "increased likelihood of survival" means an increase in PFS and/or OS in a treated individual relative to an untreated individual or relative to a control treatment regimen (e.g., a treatment regimen used in standard care for a type of cancer).

The term "treatment" or "alleviation" refers to a therapeutic measure to cure, slow down the progression of, reduce the symptoms of, and/or halt the progression of a diagnosed pathological condition or disorder. Accordingly, those in need of treatment comprise those diagnosed with or suspected of having the disorder. In certain embodiments, a cancer is successfully "treated" in an individual according to the method of the present invention if the patient exhibits one or more of the following: a reduction in the number or complete absence of cancer cells; a reduction in tumor burden; inhibition or absence of invasion of cancer cells into surrounding organs, such as spread of cancer into soft tissue and bone; inhibition or absence of tumor metastasis; inhibition or absence of tumor growth; relief of one or more symptoms associated with a specific cancer; decrease in incidence and mortality; improvement in quality of life; reduction in tumorigenesis, tumorigenesis frequency, or tumorigenesis capacity of tumors; reduction in the number or frequency of cancer stem cells in tumors; differentiation of tumorigenic cells into a non-tumorigenic state; increase in progression-free survival (PFS), disease-free survival (DFS) or overall survival (OS), duration of remission (DOR), disease control rate (DCR), complete remission (CR), partial remission (PR), stable disease (SD), delayed progressive disease (PD), prolonged time to progression (TTP), or any combination thereof.

Preventive or prophylactic measures refer to measures that prevent and/or slow down the development of a target pathological condition or disorder. Accordingly, persons in need of preventive or prophylactic measures comprise persons prone to suffering from the condition and persons to whom the condition is to be prevented.

It will be understood that when an embodiment is described herein with the language "comprising", similar embodiments described with "consisting of" and/or "consisting essentially of" are also provided.

Unless expressly stated otherwise, all ranges recited herein are inclusive; that is, a range comprises the values at the upper and lower limits of the range and all values between them. As examples, the numerical values provided by the present invention or "about" may comprise variations of ±1%, ±2%, ±3%, ±4%, ±5%, ±10%, ±15% and ±20%, and numerical equivalents thereof. All ranges are also intended to comprise all comprised subranges, although not necessarily expressly stated. For example, a range of 14 to 21 days is intended to comprise 14, 15, 16, 17, 18, 19, 20, and 21 days. Furthermore, as used herein, the term "or" indicates alternatives that may be combined where appropriate; that is, the term "or" comprises each of alternatives listed individually as well as any combinations thereof.

Unless otherwise defined, all technical and scientific terms used herein have the same meaning as commonly understood by one of ordinary skill in the art to which this disclosure relates. In case of conflict, the description (including definitions) herein shall prevail. Throughout the description and claims, the term "comprise" (or variation thereof, such as "comprises" or "comprising") will be understood to imply the inclusion of the integer or group of integers, but not the exclusion of any other integer or group of integers. Unless the context otherwise requires, singular terms shall comprise the plural, and plural terms shall comprise the singular. Any examples following "for example" or "such as" are not meant to be exhaustive or limiting.

Exemplary methods and materials are described herein, although methods and materials similar or equivalent to those described herein can also be used in the practice or testing of the present disclosure. The materials, methods, and examples are illustrative only and not restrictive.

### Sequence Information

The sequence information involved in the present invention is provided as follows:

| SEQ ID No.: | Description | Sequence information |
|---|---|---|
| 1 | HCDR-1 of the anti-TROP-2 antibody | NYGMN |
| 2 | HCDR-2 of the anti-TROP-2 antibody | WINTYTGEPTYTDDFKG |
| 3 | HCDR-3 of the anti-TROP-2 antibody | GGFGSSYWYFDV |
| 4 | LCDR-1 of the anti-TROP-2 antibody | KASQDVSIAVA |
| 5 | LCDR-2 of the anti-TROP-2 antibody | SASYRYT |
| 6 | LCDR-3 of the anti-TROP-2 antibody | QQHYITPLT |
| 7 | HCDR-1 of the anti-PD-L1 antibody | GFSLSNYD |
| 8 | HCDR-2 of the anti-PD-L1 antibody | IWTGGAT |
| 9 | HCDR-3 of the anti-PD-L1 antibody | VRDSNYRYDEPFTY |
| 10 | LCDR-1 of the anti-PD-L1 antibody | QSIGTN |
| 11 | LCDR-2 of the anti-PD-L1 antibody | YAS |
| 12 | LCDR-3 of the anti-PD-L1 antibody | QQSNSWPYT |
| 13 | heavy chain of the anti-TROP-2 antibody | QVQLQQSGSELKKPGASVKVSCKASGY TFTNYGMNWVKQAPGQGLKWMGWINT YTGEPTYTDDFKGRFAFSLDTSVSTAYL QISSLKADDTAVYFCARGGFGSSYWYFD VWGQGSLVTVSSASTKGPSVFPLAPSSK STSGGTAALGCLVKDYFPEPVTVSWNSG ALTSGVHTFPAVLQSSGLYSLSSVVTVPS SSLGTQTYICNVNHKPSNTKVDKRVEPK SCDKTHTCPPCPAPELLGGPSVFLFPPKP KDTLMISRTPEVTCVVVDVSHEDPEVKF NWYVDGVEVHNAKTKPREEQYNSTYR VVSVLTVLHQDWLNGKEYKCKVSNKAL PAPIEKTISKAKGQPREPQVYTLPPSREE MTKNQVSLTCLVKGFYPSDIAVEWESNG QPENNYKTTPPVLDSDGSFFLYSKLTVD KSRWQQGNVFSCSVMHEALHNHYTQKS LSLSPGK |
| 14 | light chain of the anti-TROP-2 antibody | DIQLTQSPSSLSASVGDRVSITCKASQDV SIAVAWYQQKPGKAPKLLIYSASYRYTG VPDRFSGSGSGTDFTLTISSLQPEDFAVY YCQQHYITPLTFGAGTKVEIKRTVAAPS VFIFPPSDEQLKSGTASVVCLLNNFYPRE AKVQWKVDNALQSGNSQESVTEQDSKD STYSLSSTLTLSKADYEKHKVYACEVTH QGLSSPVTKSFNRGEC |
| 15 | VH of the anti-PD-L1 antibody | QVQLQESGPGLVKPSETLSITCTVSGFSL SNYDISWIRQPPGKGLEWLGVIWTGGAT NYNPALKSRLTISRDNSKNQVSLKMSSV TAADTAVYYCVRDSNYRYDEPFTYWGQ GTLVTVSS |
| 16 | VL of the anti-PD-L1 antibody | EIVLTQSPDTLSVTPKEKVTLTCRASQSIG TNIHWFQQKPGQSPKLLIKYASESISGVP SRFSGSGSGTDFTLTINSVEAEDAATYYC QQSNSWPYTFGQGTKLEIK |

### Specific Models for Carrying Out the Invention

It should be understood that the examples and embodiments described herein are for illustrative purposes only, and those skilled in the art will propose various modifications or changes based on these examples and embodiments, and these modifications and changes are within the principles and scope of the present application.

Example 1: Treatment of patients with EGFR wild-type and ALK rearrangements-negative locally advanced/metastatic non-small cell lung cancer who previously neither have received immune checkpoint inhibitor treatment nor have received or at most received first-line systemic chemotherapy regimen by using anti-TROP-2 antibody-drug conjugate combined with anti-PD-L1 antibody

### 1. Combination of tested drugs:

(1) Anti-TROP-2 antibody-drug conjugate: The anti-TROP-2 antibody-drug conjugate used in this example was BT001021, which was prepared according to the method described in Example 32 of WO2019114666.
(2) Anti-PD-L1 antibody: The PD-L1 antibody used in this example was 5C10H2L2-IgG1mt, which was prepared according to the method described in Examples 1-4 and 15 of WO2017148424.

Wherein, the heavy chain variable region sequence of 5C10H2L2-IgG1mt was set forth in SEQ ID No.: 15, the light chain variable region was set forth in SEQ ID No.: 16, the light chain constant region was Ig kappa chain C region, ACCESSION: P01834, the heavy chain constant region was Ig gamma-1 chain C region, and the amino acid residues 234, 235 and 237 (EU numbering system) of ACCESSION: P01857 were mutated as follows: L234A, L235A, G237A.

### 2. Administration method

Anti-TROP-2 antibody-drug conjugate: With every 2 or 3 weeks as an administration cycle, the anti-TROP-2 antibody-drug conjugate was administered by intravenous infusion at a dose of 4 mg/kg or 5 mg/kg on the first day of each cycle;
Anti-PD-L1 antibody: With every 2 weeks or 3 weeks as an administration cycle, the anti-PD-L1 antibody was administered by intravenous infusion at a dose of 900 mg or 1200 mg on the first day of each cycle;
On the first day of each cycle, the anti-TROP-2 antibody-drug conjugate and the anti-PD-L1 antibody were administered sequentially according to the above-mentioned administration method.

### 3. Efficacy evaluation

Primary study endpoints: incidence and severity of adverse events (AEs) and objective remission rate (ORR)
Secondary study endpoints: progression-free survival (PFS), duration of remission (DOR), disease control rate (DCR), overall survival (OS), pharmacokinetic characteristics and immunogenicity.

### 4. Results

A 69-year-old man was diagnosed with EGFR wild-type and ALK rearrangements-negative non-small cell lung cancer, and the lung cancer involved multiple metastases in the mediastinum, abdominal cavity and retroperitoneal lymph nodes, liver, bone, etc. The patient previously had not received systemic treatment. The anti-TROP-2 antibody-drug conjugate of the present invention at a dose of 5 mg/kg and the anti-PD-L1 antibody at a dose of 1200 mg were administered in combination once every three weeks. Partial remission of the disease was observed after six weeks of treatment, and remission was ongoing. After multiple efficacy evaluations, the total volume reduction rate of target lesions reached 61.3%.

Example 2: Treatment of patients with EGFR wild-type and ALK rearrangements-negative locally advanced/metastatic non-small cell lung cancer who previously neither have received immune checkpoint inhibitor treatment nor have received or at most received first-line systemic chemotherapy regimen by using anti-TROP-2 antibody-drug conjugate combined with anti-PD-L1 antibody and Cisplatin/Carboplatin
1. Combination of tested drugs: The anti-TROP-2 antibody-drug conjugate and anti-PD-L1 antibody used in this example were the same as those in Example 1. Cisplatin and Carboplatin were commercially available drugs.
2. Administration method
   Anti-TROP-2 antibody-drug conjugate: With every 3 weeks as an administration cycle, the anti-TROP-2 antibody-drug conjugate was administered by intravenous infusion at a dose of 4 mg/kg or 5 mg/kg on the first day of each cycle;
   Anti-PD-L1 antibody: With every 3 weeks as an administration cycle, the anti-PD-L1 antibody was administered by intravenous infusion at a dose of 1200 mg on the first day of each cycle;
   Carboplatin: With every 3 weeks as an administration cycle, Carboplatin was administrated by intravenous infusion at a dose of AUC 5 mg/ml/min on the first day of each cycle;
   Cisplatin: With every 3 weeks as an administration cycle, Cisplatin was administered by intravenous infusion at a dose of 75 mg/m² on the first day of each cycle.
   On the first day of each cycle, according to the above administration method, the anti-TROP-2 antibody-drug conjugate, anti-PD-L1 antibody and Carboplatin were administered sequentially, or the anti-TROP-2 antibody-drug conjugate, anti-PD-L1 antibody and Cisplatin were administered sequentially.
3. Efficacy evaluation
   Primary study endpoints: incidence and severity of adverse events (AEs) and objective remission rate (ORR)
   Secondary study endpoints: progression-free survival (PFS), duration of remission (DOR), disease control rate (DCR), overall survival (OS), pharmacokinetic characteristics and immunogenicity.
4. Results
   A 34-year-old man was diagnosed with EGFR wild-type and ALK rearrangements-negative non-small cell lung cancer, and the lung cancer involved multiple metastases in the bilateral supraclavicular fossa, mediastinum and portal area, retroperitoneal lymph nodes, adrenal glands, bones, brain, etc. The patient previously had not received systemic treatment. The anti-TROP-2 antibody-drug conjugate of the present invention at a dose of 5 mg/kg, the anti-PD-L1 antibody at a dose of 1200 mg and Carboplatin at a dose of AUC 5 mg/ml/min were administered in combination once every three weeks. After 6 weeks of the administration, it was observed that the disease was stable, and the total volume of target lesions was reduced by 16.4%.

Example 3: Treatment of patients with ALK rearrangements-negative non-small cell lung cancer harboring EGFR mutation who previously have received but been failed by EGFR tyrosine kinase inhibitor (TKI) treatment by using anti-TROP-2 antibody-drug conjugate combined with anti-PD-L1 antibody and Cisplatin/Carboplatin
1. Combination of tested drugs: The anti-TROP-2 antibody-drug conjugate and anti-PD-L1 antibody used in this example were the same as those in Example 1. Cisplatin or Carboplatin were commercially available drugs.
2. Administration method
   Anti-TROP-2 antibody-drug conjugate: With every 3 weeks as an administration cycle, the anti-TROP-2 antibody-drug conjugate was administered by intravenous infusion at a dose of 4 mg/kg or 5 mg/kg on the first day of each cycle;
   Anti-PD-L1 antibody: With every 3 weeks as an administration cycle, the anti-PD-L1 antibody was administered by intravenous infusion at a dose of 1200 mg on the first day of each cycle;
   Carboplatin: With every 3 weeks as an administration cycle, Carboplatin was administered by intravenous infusion at a dose of AUC 5 mg/ml/min on the first day of each cycle;
   Cisplatin: With every 3 weeks as an administration cycle, Cisplatin was administered by intravenous infusion at a dose of 75 mg/m² on the first day of each cycle.
   On the first day of each cycle, according to the above administration method, the anti-TROP-2 antibody-drug conjugate, the anti-PD-L1 antibody and Carboplatin were administered sequentially, or the anti-TROP-2 antibody-drug conjugate, the anti-PD-L1 antibody and Cisplatin were administered sequentially.
3. Efficacy evaluation
   Primary efficacy endpoints: incidence and severity of adverse events (AEs) and objective remission rate (ORR)
   Secondary efficacy endpoints: progression-free survival (PFS), duration of remission (DOR), disease control rate (DCR), overall survival (OS), pharmacokinetic characteristics and immunogenicity.
4. Results
   A 40-year-old woman was diagnosed with non-small cell lung cancer harboring EGFR mutation. The lung cancer involved multiple metastases in the mediastinum, lung hilar, para-aortic and cardiophrenic angle region lymph nodes, pleura, liver, brain, etc. The patient previously had received but been failed by the treatment of Gefitinib combined with Anlotinib, and JM101 (EGFR monoclonal antibody) combined with Osimertinib. The anti-TROP-2 antibody-drug conjugate of the present invention at a dose of 5mg/kg, the anti-PD-L1 antibody at a dose of 1200mg, and Carboplatin at a dose of AUC 5mg/ml/min were administered once every three weeks. After 6 weeks of treatment, it was observed that the disease was stable, and the total volume of target lesions was reduced by 21.7%.

Example 4: Treatment of patients with unresectable locally advanced, recurrent or metastatic triple-negative breast cancer who previously have not received systemic therapy by using anti-TROP-2 antibody-drug conjugate combined with or without anti-PD-L1 antibody
1. Tested drugs: The anti-TROP-2 antibody-drug conjugate and anti-PD-L1 antibody used in this example were the same as in Example 1.
2. Administration method
   Anti-TROP-2 antibody-drug conjugate: With every 2 weeks or every 3 weeks as an administration cycle, the anti-TROP-2 antibody-drug conjugate was administered by intravenous infusion at a dose of 4 mg/kg or 5 mg/kg on the first day of each cycle;
   Anti-PD-L1 antibody: With every 2 weeks or every 3 weeks as an administration cycle, the PD-L1 antibody was administered by intravenous infusion at a dose of 900 mg or 1200 mg on the first day of each cycle.
   On the first day of each cycle, the anti-TROP-2 antibody-drug conjugates and anti-PD-L1 antibody were administered sequentially according to the above-mentioned administration method.
3. Efficacy evaluation
   Primary efficacy endpoints: incidence and severity of adverse events (AEs) and objective remission rate (ORR)
   Secondary efficacy endpoints: progression-free survival (PFS), duration of remission (DOR), disease control rate (DCR), overall survival (OS), pharmacokinetic characteristics and immunogenicity.
4. Results
   A 43-year-old woman was diagnosed with triple-negative breast cancer, and the breast cancer involved lung metastasis. The patient previously had received adjuvant chemotherapy with cyclophosphamide combined with epirubicin, and had progression more than 12 months after the last chemotherapy. The anti-TROP-2 antibody-drug conjugate of the present invention at a dose of 5 mg/kg and the anti-PD-L1 antibody at a dose of 900 mg were administered once every two weeks. Partial remission was observed after 6 weeks of the treatment, and the total volume of target lesions was reduced by 34.4%.

The above are only preferred examples of the present invention and are not intended to limit the present invention. Any modifications, equivalent substitutions, improvements and so on made within the spirit and principles of the present invention shall fall within the protection scope of the present invention. In addition, the technical solutions between the various examples can be combined with each other, but it must be based on the realization by those of ordinary skill in the art; when a combination of technical solutions is contradictory or cannot be realized, it should be considered that such a combination of technical solutions does not exist, and is also not within the protection scope of the present invention.

## Claims

1. Use of an anti-TROP-2 antibody-drug conjugate and an additional therapeutic agent in the manufacture of a medicament for treating a cancer in a subject, wherein the additional therapeutic agent is one or more selected from the group consisting of an anti-PD-L1 antibody or an antigen-binding fragment thereof and a chemotherapeutic agent;
the anti-TROP-2 antibody-drug conjugate comprises a camptothecin drug and an anti-TROP-2 antibody or an antigen-binding fragment thereof; the anti-TROP-2 antibody or antigen-binding fragment thereof comprises a heavy chain variable region and a light chain variable region, wherein the heavy chain variable region comprises a HCDR1 with the sequence as set forth in SEQ ID No.: 1 or a variant thereof, a HCDR2 with the sequence as set forth in SEQ ID No.: 2 or a variant thereof, and a HCDR3 with the sequence as set forth in SEQ ID No.: 3 or a variant thereof, and the light chain variable region comprises a LCDR1 with the sequence as set forth in SEQ ID No.: 4 or a variant thereof, a LCDR2 with the sequence as set forth in SEQ ID No.: 5 or a variant thereof, and a LCDR3 with the sequence as set forth in SEQ ID NO.6 or a variant thereof;
wherein, the variant has a substitution, deletion or addition of one or more amino acids (e.g., a substitution, deletion or addition of 1, 2 or 3 amino acids) as compared to the sequence from which it is derived.

2. The use according to claim 1, wherein the anti-PD-L1 antibody or antigen-binding fragment thereof comprises a heavy chain variable region and a light chain variable region, wherein the heavy chain variable region comprises a HCDR1 with the sequence as set forth in SEQ ID No.: 7 or a variant thereof, a HCDR2 with the sequence as set forth in SEQ ID No.: 8 or a variant thereof, and a HCDR3 with the sequence as set forth in SEQ ID No.: 9 or a variant thereof, and the light chain variable region comprises a LCDR1 with the sequence as set forth in SEQ ID No.: 10 or a variant thereof, a LCDR2 with the sequence as set forth in SEQ ID No.: 11 or a variant thereof, and a LCDR3 with the sequence as set forth in SEQ ID No.: 12 or a variant thereof.

3. The use according to any one of claims 1 to 2, wherein the substitution is a conservative substitution.

4. The use according to any one of claims 1 to 3, wherein the antibody or antigen-binding fragment further comprises a framework region derived from a human or murine immunoglobulin.

5. The use according to any one of claims 1 to 4, wherein the chemotherapeutic agent is selected from platinum drugs.

6. The use according to claim 5, wherein the platinum drug is selected from the group consisting of Cisplatin, Carboplatin, Sulfatodiaminocyctohexane Platin, Nedaplatin, Oxaliplatin, Lobaplatin, Satraplatin, Miboplatin, Enloplatin, Iproplatin, and Dicycloplatin.

7. The use according to any one of claims 1 to 6, wherein the antibody-drug conjugate has the following chemical structure: 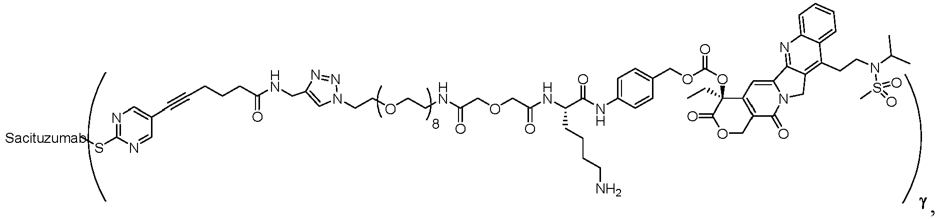 wherein γ represents the average number of drug-linker units conjugated to each antibody molecule in the antibody-drug conjugate, and the average number is in the range of 6 to 10.

8. The use according to any one of claims 1 to 7, wherein the average number of γ is between 6 and 9.

9. The use according to claim 8, wherein the average number of γ is between 6 and 8.

10. The use according to claim 9, wherein the average number of γ is about 6.1, 6.2, 6.3, 6.4, 6.5, 6.6, 6.7, 6.8, 6.9, 7, 7.1, 7.2, 7.3, 7.4, 7.5, 7.6, 7.7, 7.8 or 7.9.

11. The use according to any one of claims 1 to 10, wherein the cancer is a solid tumor or a non-solid tumor.

12. The use according to any one of claims 1 to 11, wherein the cancer is selected from the group consisting of esophageal cancer, brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor, prostate cancer, and thyroid cancer.

13. The use according to any one of claims 1 to 12, wherein the cancer is selected from the group consisting of lung cancer, breast cancer and ovarian cancer.

14. The use according to claim 13, wherein the cancer is selected from the group consisting of non-small cell lung cancer (NSCLC), triple negative breast cancer (TNBC), and epithelial ovarian cancer.

15. The use according to claim 13, wherein the breast cancer is selected from the group consisting of triple negative breast cancer, HR+/HER2- breast cancer, HR-/HER2+ breast cancer, HR+/HER2+ breast cancer, and BRCA+ breast cancer.

16. The use according to claim 14, wherein the non-small cell lung cancer is selected from the group consisting of EGFR wild-type or EGFR-mutated non-small cell lung cancer.

17. The use according to claim 14, wherein the non-small cell lung cancer is an advanced/metastatic non-small cell lung cancer.

18. The use according to claim 14, wherein the non-small cell lung cancer is an ALK rearrangements-negative non-small cell lung cancer.

19. The use according to claim 16, wherein the EGFR-mutated non-small cell lung cancer is advanced/metastatic, and the EGFR mutation is single mutation or compound mutation.

20. The use according to any one of claims 1 to 19, wherein the antibody-drug conjugate, anti-PD-L1 antibody or antigen-binding fragment thereof, and Carboplatin or Cisplatin have a cycle of administration of 14 to 42 days, for example, 21 to 42 days.

21. The use according to claim 20, wherein the cycle of administration is 21 days, 28 days or 42 days.

22. The use according to any one of claims 1 to 21, wherein the antibody-drug conjugate is administered at a dosage of 1 mg/kg to 10 mg/kg per time.

23. The use according to any one of claims 1 to 22, wherein the antibody-drug conjugate is administered at a dose selected from the group consisting of 1 mg/kg, 2 mg/kg, 3 mg/kg, 4 mg/kg, 5 mg/kg, 6mg/kg, 7mg/kg, 8mg/kg, 9mg/kg, and 10mg/kg per time.

24. The use according to any one of claims 1 to 23, wherein the anti-PD-L1 antibody or antigen-binding fragment thereof is administered at a dose of 900 mg to 1500 mg per time.

25. The use according to claim 24, wherein the anti-PD-L1 antibody or antigen-binding fragment thereof is administered at a dose selected from the group consisting of 900 mg, 1000 mg, 1100 mg, 1200 mg, 1300 mg, 1400 mg, and 1500 mg per time.

26. The use according to any one of claims 1 to 25, wherein the Carboplatin is administered at a dose, calculated as area under curve (AUC), of 1 mg/ml/min to 10 mg/ml/min per time; or, the Cisplatin is administered at a dose of 50 mg/m² to 150 mg/m² per time.

27. The use according to claim 26, wherein Carboplatin is administered at a dose, calculated as area under curve (AUC), selected from the group consisting of 1 mg/ml/min, 2 mg/ml/min, and 2.5 mg/ml/min, 3mg/ml/min, 3.75 mg/ml/min, 4mg/ml/min, 5mg/ml/min, 6mg/ml/min, 7mg/ml/min, and 8mg/ml/min per time; or, Cisplatin is administered at a dose selected from the group consisting of 50 mg/m², 75 mg/m², 100 mg/m², 120 mg/m², and 150 mg/m² per time.

28. A method for treating a cancer in a subject, comprising a step of administering to the subject a therapeutically effective amount of a combination of an anti-TROP-2 antibody-drug conjugate and an additional therapeutic agent, wherein the additional therapeutic agent is one or more selected from the group consisting of an PD-L1 antibody or an antigen-binding fragment thereof and chemotherapeutic agent;
the anti-TROP-2 antibody-drug conjugate comprises a camptothecin drug and an anti-TROP-2 antibody or an antigen-binding fragment thereof, the anti-TROP-2 antibody or antigen-binding fragment thereof comprises a heavy chain variable region and a light chain variable region, wherein the heavy chain variable region comprises a HCDR1 with the sequence as set forth in SEQ ID No.: 1 or a variant thereof, a HCDR2 with the sequence as set forth in SEQ ID No.: 2 or a variant thereof, and a HCDR3 with the sequence as set forth in SEQ ID No.: 3 or a variant thereof, and the light chain variable region comprises a LCDR1 with the sequence as set forth in SEQ ID No.: 4 or a variant thereof, a LCDR2 with the sequence as set forth in SEQ ID No.: 5 or a variant thereof, and a LCDR3 with the sequence as set forth in SEQ ID No.: 6 or a variant thereof;
wherein, the variant has a substitution, deletion or addition of one or more amino acids (e.g., a substitution, deletion or addition of 1, 2 or 3 amino acids) as compared to the sequence from which it is derived.

29. The method according to claim 28, wherein the anti-PD-L1 antibody or antigen-binding fragment thereof comprises a heavy chain variable region and a light chain variable region, wherein the heavy chain variable region comprises a HCDR1 with the sequence as set forth in SEQ ID NO.:7 or a variant thereof, a HCDR2 with the sequence as set forth in SEQ ID No.: 8 or a variant thereof, and a HCDR3 with the sequence as set forth in SEQ ID No.: 9 or a variant thereof, and the light chain variable region comprises a LCDR1 with the sequence as set forth in SEQ ID No.: 10 or a variant thereof, a LCDR2 with the sequence as set forth in SEQ ID No.: 11 or a variant thereof, and a LCDR3 with the sequence as set forth in SEQ ID No.: 12 or a variant thereof.

30. The method according to any one of claims 28 to 29, wherein the substitution is a conservative substitution.

31. The method according to any one of claims 28 to 30, wherein the antibody or antigen-binding fragment further comprises a framework region derived from a human or murine immunoglobulin.

32. The method according to any one of claims 28 to 31, wherein the chemotherapeutic agent is selected from platinum drugs.

33. The method according to claim 32, wherein the platinum drug is selected from the group consisting of Cisplatin, Carboplatin, Sulfatodiaminocyctohexane Platin, Nedaplatin, Oxaliplatin, Lobaplatin, Satraplatin, Miboplatin, Enloplatin, Iproplatin, and Dicycloplatin.

34. The method according to any one of claims 28 to 33, wherein the antibody-drug conjugate has the following chemical structure: 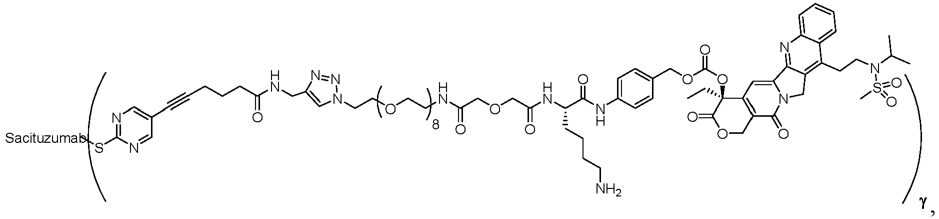 wherein γ represents the average number of drug-linker units conjugated to each antibody molecule in the antibody-drug conjugate, and the average number is in the range of 6 to 10.

35. The method according to any one of claims 28 to 34, wherein the average number of γ is between 6 and 9.

36. The method according to claim 35, wherein the average number of γ is between 6 and 8.

37. The method according to claim 35, wherein the average number of γ is about 6.1, 6.2, 6.3, 6.4, 6.5, 6.6, 6.7, 6.8, 6.9, 7, 7.1, 7.2, 7.3, 7.4, 7.5, 7.6, 7.7, 7.8 or 7.9.

38. The method according to any one of claims 28 to 37, wherein the cancer is a solid tumor or a non-solid tumor.

39. The method according to any one of claims 28 to 38, wherein the cancer is selected from the group consisting of esophageal cancer, brain tumor, lung cancer, squamous cell carcinoma, bladder cancer, gastric cancer, ovarian cancer, peritoneal cancer, pancreatic cancer, breast cancer, head and neck cancer, cervical cancer, endometrial cancer, colorectal cancer, liver cancer, kidney cancer, non-Hodgkin's lymphoma, central nervous system tumor, prostate cancer and thyroid cancer.

40. The method according to any one of claims 28 to 39, wherein the cancer is selected from the group consisting of lung cancer, breast cancer and ovarian cancer.

41. The method according to claim 40, wherein the cancer is selected from the group consisting of non-small cell lung cancer (NSCLC), triple negative breast cancer (TNBC), and epithelial ovarian cancer.

42. The method according to claim 40, wherein the breast cancer is selected from the group consisting of triple negative breast cancer, HR+/HER2- breast cancer, HR-/HER2+ breast cancer, HR+/HER2+ breast cancer, and BRCA+ breast cancer.

43. The method according to claim 41, wherein the non-small cell lung cancer is selected from the group consisting of EGFR wild-type or EGFR-mutated non-small cell lung cancers.

44. The method according to claim 41, wherein the non-small cell lung cancer is selected from the group consisting of advanced/metastatic non-small cell lung cancer.

45. The method according to claim 41, wherein the non-small cell lung cancer is an ALK rearrangements-negative non-small cell lung cancer.

46. The method according to claim 43, wherein the EGFR-mutated non-small cell lung cancer is advanced/metastatic, and the mutation of EGFR is single mutation or compound mutation.

47. The method according to any one of claims 28 to 46, wherein the antibody-drug conjugate, anti-PD-L1 antibody or antigen-binding fragment thereof, and Carboplatin or Cisplatin have a cycle of administration of 14 to 42 days, for example 21 to 42 days.

48. The method according to claim 47, wherein the cycle of administration is 21 days, 28 days or 42 days.

49. The method according to any one of claims 28 to 48, wherein the antibody-drug conjugate is administered at a dose of 1 mg/kg to 10 mg/kg per time.

50. The method according to any one of claims 28 to 49, wherein the antibody-drug conjugate is administered at a dose selected from the group consisting of 1 mg/kg, 2 mg/kg, 3 mg/kg, 4 mg/kg, 5 mg/kg, 6mg/kg, 7mg/kg, 8mg/kg, 9mg/kg, and 10mg/kg per time.

51. The method according to any one of claims 28-50, wherein the anti-PD-L1 antibody or antigen-binding fragment thereof is administered at a dose of 900 mg to 1500 mg per time.

52. The method according to claim 51, wherein the anti-PD-L1 antibody or antigen-binding fragment thereof is administered at a dose selected from the group consisting of 900 mg, 1000 mg, 1100 mg, 1200 mg, 1300 mg, 1400 mg, and 1500 mg per time.

53. The method according to any one of claims 28 to 52, wherein the Carboplatin is administered at a dose, calculated as area under curve (AUC), of 1 mg/ml/min to 10 mg/ml/min per time; or, the Cisplatin is administered at a dose of 50 mg/m² to 150 mg/m² per time.

54. The method according to claim 53, wherein Carboplatin is administered at a dose, calculated as area under curve (AUC), selected from the group consisting of 1 mg/ml/min, 2 mg/ml/min, and 2.5 mg/ml/min, 3mg/ml/min, 3.75 mg/ml/min, 4mg/ml/min, 5mg/ml/min, 6mg/ml/min, 7mg/ml/min, and 8mg/ml/min per time; or, Cisplatin is administered at a dose selected from the group consisting of 50 mg/m², 75 mg/m², 100 mg/m², 120 mg/m², and 150 mg/m² per time.

55. The method according to any one of claims 28 to 54, wherein the subject is a patient with non-small cell lung cancer who previously neither has received an immune checkpoint inhibitor therapy nor has received or at most received a first-line systemic chemotherapy regimen; or the subject is a patient with non-small cell lung cancer who previously has received an adjuvant, neoadjuvant or radical chemoradiotherapy, but the cancer progresses during the therapy or within 6 months after the end of the therapy; or the subject is a patient with non-small cell lung cancer who has received but been failed by an EGFR kinase inhibitor therapy; or the subject is a patient with non-small cell lung cancer who previously neither has received a systemic anti-tumor therapy for locally advanced or metastatic non-small cell lung cancer nor has received an immune checkpoint inhibitor therapy; or the subject is a patient with unresectable locally advanced, recurrent or metastatic triple-negative breast cancer who previously has not received a systemic therapy.

56. The method according to any one of claims 28 to 55, which reduces tumor size by at least about 10%.

57. A kit, which comprises an anti-TROP-2 antibody-drug conjugate and an additional therapeutic agent, wherein the additional therapeutic agent is one or more selected from the group consisting of an anti-PD-L1 antibody or an antigen-binding fragment thereof and a chemotherapeutic agent, wherein the anti-TROP-2 antibody-drug conjugate, anti-PD-L1 antibody or antigen-binding fragment thereof, and chemotherapeutic agent are as defined in any one of claims 1 to 56.

58. A pharmaceutical composition, which comprises an anti-TROP-2 antibody-drug conjugate and an additional therapeutic agent, wherein the additional therapeutic agent is one or more selected from the group consisting of an anti-PD-L1 antibody or an antigen-binding fragment thereof and a chemotherapeutic agent, and the anti-TROP-2 antibody-drug conjugate, anti-PD-L1 antibody or antigen-binding fragment thereof, and chemotherapeutic agent are as defined in any one of claims 1 to 56.

59. The composition according to claim 58, further comprising one or more pharmaceutically acceptable excipients, diluents or carriers.
